# FASCICULE DE BREVET EUROPEEN

(11) **EP 0 984 920 B1**
(45) Date de publication et mention de la délivrance du brevet: **16.07.2003**
(21) Numéro de dépôt: 98928359.3
(22) Date de dépôt: 26.05.1998
(51) Int. Cl.: C07C 213/02

(54) **NOUVEAU PROCEDE DE PREPARATION DE 2-TRIFLUORO-METHOXY-ANILINE**
VERFAHREN ZUR HERSTELLUNG VON 2-TRIFLUORMETHOXYNALIN
NOVEL METHOD FOR PREPARING 2-TRIFLUORO-METHOXY-ANILINE

(30) Priorité: 27.05.1997 FR 9706483
(43) Date de publication de la demande: 15.03.2000
(73) Titulaire: RHODIA CHIMIE, 92408 Courbevoie Cédex (FR)
(72) Inventeur: DURY, Michel, F-69006 Lyon (FR)
(74) Mandataire: Jacobson, Claude
(86) Numéro de dépôt international: FR9801050
(87) Numéro de publication internationale: WO98054121

(56) Documents cités:
- EP-A- 0 546 391
- CHEMICAL ABSTRACTS, vol. 55, no. 23, 1961 Columbus, Ohio, US; abstract no. 23408e, L.M. YAGUPOLSKII: "Synthesis of derivatives of phenyl trifluoromethyl ether." XP002056847 cité dans la demande & ZHUR. OBSHCHEI KHIM, vol. 32, 1961, pages 915-924,
- W.A. SHEPPARD: "alpha-Fluorinated ethers. I. Aryl fluoroalkyl ethers" JOURNAL OF ORGANIC CHEMISTRY., vol. 29, no. 1, 1964, pages 1-11, XP002056846 EASTON US cité dans la demande

## Description

L'invention concerne un procédé de préparation de composés aromatiques comportant un groupe 2-méthoxy amino-phényle, plus particulièrement la 2-trifluorométhoxy-aniline.

La trifluorométhoxy-2-aniline est un intermédiaire de la synthèse de nombreux produits pharmaceutiques et agrochimiques.

Différentes voies de synthèses ont été proposées.

On connaît ainsi plusieurs procédés mettant en oeuvre le 2-nitrotrifluorométhoxyphényle et consistant à réduire celui-ci par hydrogénation catalytique ou par SnCl₂ en milieu acide (ZHUR.OBSHCHEI.KHIM. (31) 1961, p. 915-924, (CA C55) 23408); J. Org. Chem. (29), 1964, p. 1-11).

Un autre procédé décrit dans ZHUR.OBSHCHEI.KHIM. (31) 1961, p. 915-924 consiste à réduire sélectivement le 4-iodo-2-nitro-trifluorométhoxyphényle, obtenu à partir de la 2-nitro-trifluorométhoxy-aniline, pour obtenir la 4-iodo-trifluorométhoxy-aniline et à hydrogéner celle-ci sur Nickel de Ramey.

La présente invention fournit un nouveau procédé de préparation de composés aromatiques comportant un groupe 2-trifluorométhoxy amino-phényle à partir de composés aromatiques comportant un groupe 2-méthoxy amino-phényle.

Plus précisément, l'invention a pour objet un procédé de préparation d'un composé de formule I 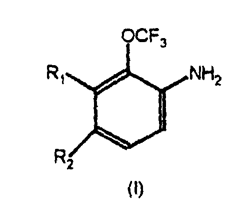 dans laquelle R₁, et R₂ identiques ou différents représentent un atome d'hydrogène, un groupe alcoyle en C₁-C₆ ou R₁ et R₂ pris ensemble avec les atomes de carbone auxquels ils sont liés représentent un groupe aryle en C₆-C₁₀ ;
caractérisé en ce qu'il comprend les étapes consistant à :
a) éventuellement faire réagir un composé de formule générale II : 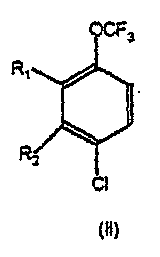 dans laquelle R₁ et R₂ représentent les valeurs ci-dessus ou R₁ représente un atome d'hydrogène ou un groupe alcoyle en C₁-C₆
   ou R₁ et R₂ pris ensemble avec l'atome de carbone auxquels ils sont liés représentent un noyau aryle en C₆-C₁₀,
   avec un réactif de nitration pour former un composé de formule III: 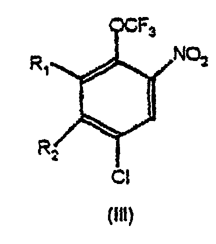 dans laquelle R₁ et R₂ sont tels que définis précédemment
b) réduire le composé de formule III pour former le composé de formule IV : 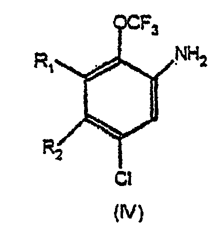 dans laquelle R₁ et R₂ sont tels que définis précédemment
c) soumettre à une hydrogénolyse le composé obtenu en b) pour obtenir le composé de formule I ; et
d) séparer le composé obtenu à l'étape c) du mélange réactionnel.

L'étape a) est réalisée avantageusement en faisant réagir le composé de formule générale II avec de l'acide nitrique en milieu acide sulfurique, dans un solvant protique, par exemple l'acide acétique ou un solvant aprotique avantageusement un solvant chloré, par exemple, le dichlorométhane ou sans solvant.

La température réactionnelle se situe dans la plage de 0 à 60°C, la réaction étant de préférence conduite entre 20 et 40°C.

A l'issue de l'étape a), on sépare le cas échéant les énantiomères de position obtenus pour isoler le composé 2-nitro trifluorométhoxy phényle.

L'étape b) est avantageusement une hydrogénation catalytique en présence de nickel de Raney dans un solvant tel qu'un alcool ou l'acide acétique.

En variante, la réduction de la fonction nitro en amino peut être réalisée selon une réaction de Béchamp en présence de Fe, en milieu acide chlorhydrique dans l'acide acétique ou dans l'eau, en tant que solvant.

Une autre variante consiste à réduire le composé de l'étape précédente, par SnCl₂ en milieu acide chlorhydrique dans l'éthanol, en chauffant le milieu réactionnel au reflux pendant une durée allant de une à plusieurs heures.

L'étape c) peut être réalisée par hydrogénation du composé de l'étape b) en présence de nickel de Raney en utilisant comme solvant un alcool ou l'acide acétique.

Les étapes b) et c) peuvent être menées simultanément.

L'étape a) est optionnelle mais préférée, toute autre méthode d'obtention du composé de formule III étant acceptable.

Les produits de départ sont des composés connus pouvant être préparés de manière simple.

Par exemple, le 4-chlorotrifluorométhoxy-benzène peut par exemple être préparée à partir du p-chloro phénol comme décrit dans ZHUR.OBSHCHEI.KHIM. (31) 1961, p. 915-924, (CA C55) 23408) ; J. Org. Chem. (29), 1964, p. 1-11.

Comme composés de formule II, on préfère ceux dans lesquels R₁ et R₂ représentent l'hydrogène

L'avantage dans ce dernier cas est la sélectivité de la réaction de l'étape a) et donc l'absence de nécessité de procéder à une étape ultérieure de séparation des isomères.

On décrira ci-après plus particutièrement la préparation de la 2-trifluorométhoxy-aniline à partir du 4-chloro-trifluorométhoxybenzène.

### EXEMPLE

### 1. Synthèse du 4-chloro-2(-3) nitro trifluorométhoxybenzène

Sur un pied de 1640 g d'acide sulfurique à 96,8 % et de 680,4 g d'acide nitrique à 100 %, préalablement chauffé à 40°C, on charge en une heure 1207,0 g de 4-chloro trifluorométhoxybenzène. La masse réactionnelle est ensuite chauffée à 50°C puis maintenue à cette température pendant quatre heures. Après arrêt de l'agitation, la phase organique supérieure est décantée à 40°C, puis coulée sur 638,2 g de soude caustique à 18,2 %. Après de nouveau décantation, la phase organique est lavée par 250 ml d'eau. La masse réactionnelle brute contient un mélange 50/50 de 4-chloro-2-nitro trifluorométhoxybenzène et de 4-chloro-3-nitro trifluorométhoxybenzène (poids: 1550 g - pureté en anilines : 91,8 %). Le rendement de nitration est de 96 %.

### 2. Synthèse du 2-amino-4-chloro trifluorométhoxybenzène

Dans un autoclave de 500 ml, on charge 10 g de nickel de Raney à 50 % d'humidité dans 170 ml de méthanol. Après avoir purgé l'autoclave par de l'hydrogène, la masse réactionnelle est chauffée à 50°C et la pression d'hydrogène est réglée sur 12-13 bar. 107,0 g de 2-nitro 4-chloro trifluorométhoxybenzène sont ensuite chargés en continu entre 1h30 et 2 heures sur la masse réactionnelle. Après l'addition du composé nitré, la masse réactionnelle est maintenue 30 minutes à 1 heure à 50°C. La masse réactionnelle de 2-amino-4-chloro trifluorométhoxybenzène ainsi formée est engagée directement à l'étape d'hydrogénolyse.

### 3. Synthèse de la 2-trifluorométhoxyaniline

Dans le réacteur précédent contenant la masse réactionnelle brute, et sous 12-13 bar d'hydrogène, on introduit 68,5 g de soude caustique à 30,2 % en 15 minutes tout en portant la température de la masse réactionnelle de 50 à 70°C. La masse réactionnelle est ensuite maintenue à 70°C pendant six heures. Après ce laps de temps, l'hydrogénolyse est terminée et la masse réactionnelle est refroidie à température ambiante puis additionnée de 100 ml d'eau. Le catalyseur est ensuite filtré et le filtrat biphasique est ensuite chauffé jusqu'à 100°C afin d'éliminer le méthanol. Le culot de décantation est ensuite décanté pour conduire à 77,7 g de 2-amino trifluorométhoxybenzène brut titrant 96,9 % (rendement chimique : 96 %). Le produit brut est ensuite distillé sous vide (64°C sous 20 torr) pour conduire au 2-amino trifluorométhoxybenzène de titre supérieur à 99 %.

Ce même mode opératoire peut être effectué sur le mélange 50/50 de 4-chloro-2-nitro trifluorométhoxybenzène et de 4-chloro-3-nitro trifluorométhoxybenzène. On obtient alors un mélange de 2-amino trifluorométhoxybenzène (2-trifluorométhoxyaniline) et de 3-amino trifluorométhoxybenzène (3-trifluorométhoxyaniline). Ces deux anilines sont alors séparées par distillation sous vide (Ebullition respective sous 2,7:10³ Pa (20 torr) : 64 et 89°C).

## Revendications

1. Procédé de préparation d'un composé de formule I 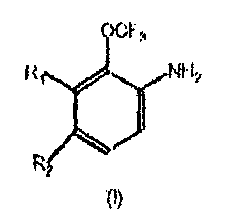 dans laquelle R₁ et R₂ identiques ou différents représentent un atome d'hydrogène, un groupe alcoyle en C₁-C₆ ou R₁ et R₂ pris ensemble avec les atomes de carbone auxquels ils sont liés représentent un groupe aryle en C₆-C₁₀ ;
**caractérisé en ce qu'**il comprend les étapes consistant à :
a) éventuellement faire réagir un composé de formule générale II : 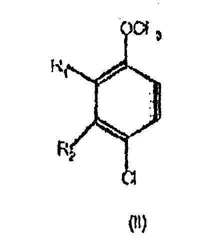 dans laquelle R₁ et R₂ sont tels que définis ci-dessus ou R₁ représente un atome d'hydrogène ou un groupe alcoyle en C₁-C₆ ou R₁ et R₂ pris ensemble avec l'atome de carbone auxquels ils sont liés représentent un noyau aryle en C₆-C₁₀, avec un réactif de nitration pour former un composé de formule III: 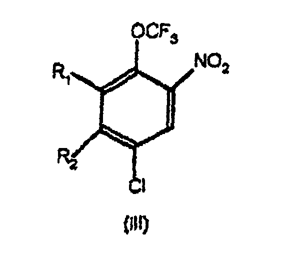 dans laquelle R₁ et R₂ sont tels que définis précédemment
b) réduire le composé de formule III pour former le composé de formule IV ; 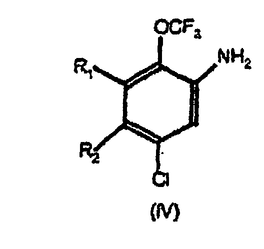 dans laquelle R₁ et R₂ sont tels que définis précédemment
c) soumettre à une hydrogénolyse le composé obtenu en b) pour obtenir le composé de formule I ; et
d) séparer le composé obtenu à l'étape c) du mélange réactionnel.

2. Procédé selon la revendication 1, **caractérisé en ce que** l'étape a) comprend la réaction du composé de formule générale Il avec de l'acide nitrique en milieu acide sulfurique dans un solvant protique ou aprotique ou sans solvant à une température comprise entre 0 et 60°C.

3. Procédé selon la revendication 1, **caractérisé en ce que** l'étape b) comprend une hydrogénation catalytique en présence de nickel de Raney.

4. Procédé selon la revendication 1, **caractérisé en ce que** l'étape c) est une hydrogénation catalytique en présence de nickel de Raney.

5. Procédé selon l'une des revendications précédentes, **caractérisé en ce que** l'on prépare la 2-trifluorométhoxy-aniline à partir du 4-chlorotrifluorométhoxy-benzène.

## Claims

1. Process for the preparation of a compound of formula I 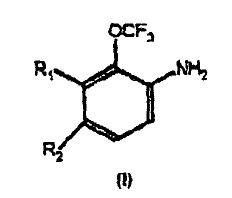 in which R₁ and R₂, which may be the same or different, represent a hydrogen atom, a C₁ to C₆ alkyl group or R₁ and R₂ taken together with the carbon atoms to which they are bonded represent a C₈-C₁₀ aryl group, **characterized in that** it comprises the stages consisting of:
a) optionally reacting a compound of general formula II: 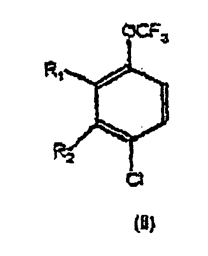 in which R₁ and R₂ are as defined hereinbefore or R₁ represents a hydrogen atom or a C₁-C₆ alkyl group or R₁ and R₂ taken together with the carbon atom to which they are bonded represent a C₈-C₁₀ aryl nucleus, with a nitrating agent for forming a compound of formula III: 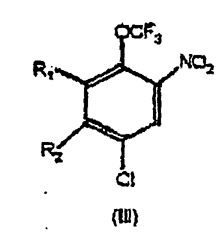 in which R₁ and R₂ are as defined hereinbefore,
b) reducing the compound of formula III to form the compound of formula IV: 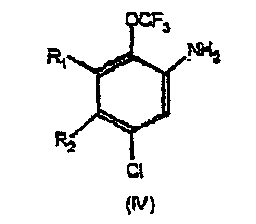 in which R₁ and R₂ are as defined hereinbefore,
c) subjecting to a hydrogenolysis the compound obtained in b) in order to obtain the compound of formula I and
d) separating the compound obtained in stage c) from the reaction mixture.

2. Process according to claim 1, characierized in that stage a) comprises the reaction of the compound of general formula II with nitric acid in a sulphuric acid medium in a protic or aprotic solvent or without a solvent at a temperature between 0 and 60°C.

3. Process according to claim 1, **characterized in that** stage b) comprises a catalytic hydrogenation in the presence of Raney nickel.

4. Process according to claim 1, **characterized in that** stage c) is a catalytic hydrogenation in the presence of Raney nickel.

5. Process according to one of the preceding claims, **characterized in that** 2-trifluoromethoxy-aniline is prepared from 4-chlorotrifluoromethoxy benzene.

## Patentansprüche

1. Verfahren zur Herstellung einer Verbindung der Formel I: 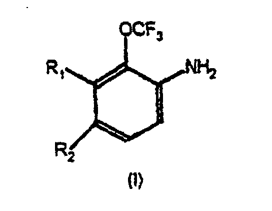 worin R₁ und R₂ gleich oder verschieden sind und ein Wasserstoffatom, eine (C₁-C₆)Alkylgruppe bedeuten oder R₁ und R₂ zusammen mit den Kohlenstoffatomen an die sie gebunden sind eine (C₆-C₁₀)Arylgruppe bilden;
**dadurch gekennzeichnet, dass** es folgende Stufen umfasst:
a) gegebenenfalls Umsetzung einer Verbindung der allgemeinen Formel II: 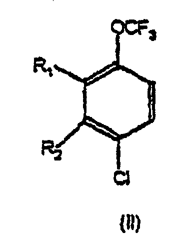 worin R₁ und R₂ wie vorstehend definiert sind oder R₁ ein Wasserstoffatom oder eine (C₁-C₆)Alkylgruppe darstellt und R₁ und R₂ zusammen mit den Kohlenstoffatomen an die sie gebunden sind einen (C₆-C₁₀)Arylrest bilden, mit einem Nitrierungs-Reagens zur Bildung einer Verbindung der Formel III: 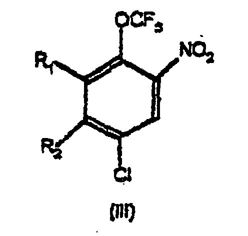 worin R₁ und R₂ wie vorstehend definiert sind,
b) Reduktion der Verbindung der Formel III unter Bildung der Verbindung der Formel IV: 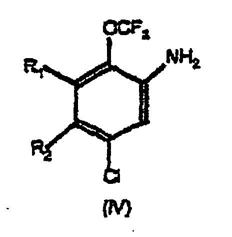 worin R₁ und R₂ wie vorstehend definiert sind,
c) Unterziehen der in b) erhaltenen Verbindung einer Hydrogenolyse zur Bildung der Verbindung der Formel I; und
d) Abtrennen der in der Stufe c) erhaltenen Verbindung aus dem Reaktionsgemisch.

2. Verfahren nach Anspruch 1, **dadurch gekennzeichnet, dass** die Stufe a) die Reaktion der Verbindung der allgemeinen Formel II mit Salpetersäure in schwefelsaurem Milieu in einem protischen oder aprotischen Lösungsmittel oder ohne Lösungsmittel bei einer Temperatur von 0 bis 60°C umfasst.

3. Verfahren nach Anspruch 1, **dadurch gekennzeichnet, dass** die Stufe b) eine katalytische Hydrierung in Anwesenheit von Raney-Nickel umfasst.

4. Verfahren nach Anspruch 1, **dadurch gekennzeichnet, dass** die Stufe c) eine katalytische Hydrierung in Anwesenheit von Raney-Nickel umfasst.

5. Verfahren nach einem der vorhergehenden Ansprüche, **dadurch gekennzeichnet, dass** man 2-Trifluormethoxyanilln ausgehend von 4-Chlortrifluormethoxybenzol herstellt.
